(19)

**Europäisches Patentamt**
**European Patent Office**
**Office européen des brevets**

(11) **EP 3 356 823 B1**

(12) **EUROPEAN PATENT SPECIFICATION**

(45) Date of publication and mention of the grant of the patent:
**22.04.2020 Bulletin 2020/17**

(51) Int Cl.:
***G01N 33/543*** *(2006.01)*

(21) Application number: **16784403.4**

(22) Date of filing: **27.09.2016**

(86) International application number:
**PCT/EP2016/072886**

(87) International publication number:
**WO 2017/055229 (06.04.2017 Gazette 2017/14)**

(54) **A MODIFIED BDD SUBSTRATE AND ITS USE AS AN IMPEDANCE SENSOR FOR DETECTION OF BIOLOGICAL MOLECULES AND A SYSTEM AND METHOD FOR MONITORING PATHOGENS**

MODIFIZIERTES BDD-SUBSTRAT UND DESSEN VERWENDUNG ALS IMPEDANZSENSOR ZUM NACHWEIS BIOLOGISCHER MOLEKÜLE UND SYSTEM UND VERFAHREN ZUR ÜBERWACHUNG VON PATHOGENEN

SUBSTRAT DE DDB MODIFIÉ, SON PROCÉDÉ DE PRÉPARATION, CAPTEUR D'IMPÉDANCE PERMETTANT LA DÉTECTION DE MOLÉCULES BIOLOGIQUES, SYSTÈME ET PROCÉDÉ DE SURVEILLANCE D'AGENTS PATHOGÈNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.09.2015 PL 41413615**
**22.03.2016 PL 41657516**
**22.03.2016 PL 41657716**

(43) Date of publication of application:
**08.08.2018 Bulletin 2018/32**

(73) Proprietor: **Sensdx Sp. z o.o.**
**02-676 Warszawa (PL)**

(72) Inventors:
• **NIDZWORSKI, Dawid**
**80-180 Gdansk (PL)**
• **WNUK, Elzbieta Magdalena**
**82-522 Sadlinki (PL)**
• **NIEDZIALKOWSKI, Pawel Lucjan**
**77-300 Czluchow (PL)**
• **OSSOWSKI, Tadeusz**
**80-241 Gdansk (PL)**
• **SIUZDAK, Katarzyna**
**80-766 Gdansk (PL)**
• **SAWCZAK, Miroslaw**
**83-332 Dzierzazno (PL)**
• **BOGDANOWICZ, Robert**
**80-287 Gdansk (PL)**

• **SOBASZEK, Michal**
**80-180 Gdansk (PL)**
• **KRYCH, Piotr**
**03-528 Warszawa (PL)**
• **KUPCZUNAS, Artur**
**62-800 Kalisz (PL)**

(74) Representative: **Witek, Rafal**
**WTS Patent Attorneys**
**Witek, Sniezko & Partners**
**ul. Weigla 12**
**53-114 Wroclaw (PL)**

(56) References cited:
• **ANCHANA PREECHAWORAPUN ET AL: "Development of Amperometric Immunosensor Using Boron-Doped Diamond with Poly( o -aminobenzoic acid)", ANALYTICAL CHEMISTRY, vol. 80, no. 6, 1 March 2008 (2008-03-01), pages 2077-2083, XP055332884, US ISSN: 0003-2700, DOI: 10.1021/ac702146u**
• **LUBOMÍR SVORC ET AL: "Doping Level of Boron-Doped Diamond Electrodes Controls the Grafting Density of Functional Groups for DNA Assays", ACS APPLIED MATERIALS AND INTERFACES, vol. 7, no. 34, 2 September 2015 (2015-09-02), pages 18949-18956, XP055332998, US ISSN: 1944-8244, DOI: 10.1021/acsami.5b06394**

**(Cont. next page)**

EP 3 356 823 B1

- AMANI CHROUDA ET AL: "Electrically addressable deposition of diazonium-functionalized antibodies on boron-doped diamond microcells for the detection of ochratoxin A", ANALYTICAL METHODS, vol. 7, no. 6, 1 January 2015 (2015-01-01) , pages 2444-2451, XP055333002, GBR ISSN: 1759-9660, DOI: 10.1039/C4AY02899F
- ARYA SUNIL K ET AL: "Label free biosensor for sensitive human influenza virus hemagglutinin specific antibody detection using coiled-coil peptide modified microelectrode array based platform", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 194, 24 December 2013 (2013-12-24), pages 127-133, XP028609190, ISSN: 0925-4005, DOI: 10.1016/J.SNB.2013.12.066
- DA-JUNG CHUNG ET AL: "One-step modification of various electrode surfaces using diazonium salt compounds and the application of this technology to electrochemical DNA (E-DNA) sensors", ELECTROCHIMICA ACTA, ELSEVIER SCIENCE PUBLISHERS, BARKING, GB, vol. 76, 10 May 2012 (2012-05-10), pages 394-403, XP028498931, ISSN: 0013-4686, DOI: 10.1016/J.ELECTACTA.2012.05.044 [retrieved on 2012-05-16]

## Description

TECHNICAL FIELD

The present disclosure relates to a modified BDD (Boron Doped Diamond) substrate, a method for preparing thereof and an ultrasensitive EIS (Electrochemical Impedance Spectroscopy) sensor, comprising the modified BDD substrate, for detection of various biological molecules, such as: amino acids, hormones, sugars, nucleic acids, peptides or proteins. For example, the sensor may be used for detection of the M1 protein of influenza virus in a biological sample, such as a sample taken from a living organism. The disclosure also relates to a system and method for monitoring pathogens.

BACKGROUND

Boron doped diamond (BDD) substrates serve as precursors of BDD electrodes. The process for production of BDD electrodes, due to their high material costs, typically comprises deposition of a thin boron doped diamond layer on a substrate of another material, for example on a silicon (Si) substrate. BDD electrodes show substantially good electrochemical properties, including a wide potential window in both aqueous and non-aqueous environments, high voltage of release of hydrogen, oxygen or chloride, low and stable background current, well-polarizable surface, high mechanical and chemical resistance, including corrosion resistance, as well as high durability and high thermal conductivity. Nonetheless, the main drawback of BDD electrodes is a weak surface adsorption, due to the aliphatic character of the BDD surface.

BDD substrates are mainly used in voltammetry techniques, where they serve as working electrodes of systems for determination whether a particular substance is present in medicaments or in food products. Following a suitable surface modification, the BDD electrode may be also used in the biological sensors that make use of biological recognition elements (also known as probes) such as DNA proteins, peptides, amino acids, hormones or sugars, which can selectively bind to the analyte being also the biological molecule able to form a specific probe-analyte binding (probe-analyte complexes).

There are known methods for modification of substrates, such as BDD substrates, aiming to implement the modified substrate as a working electrode into various electrochemical sensors.

The publications: "Diamond films: Precious biosensors" (Carlisle, J. A., Nat. Mater. 3, 668-669 (2004)) and "Influence of the boron doping level on the electrochemical oxidation of the azo dyes at Si/BDD thin film electrodes" (Bogdanowicz, R. et al., Diam. Relat. Mater. 39, 82-88 (2013)) describe experiments that involve attaching of various biomolecules of interest (including amino acids, peptides and polynucleic acid sequences) to BDD surfaces, wherein the BDD electrodes are prepared by a microwave plasma enhanced-chemical vapor deposition (MW PE CVD) method and are hydrogen-terminated (H-BDD). Therefore, the surfaces of diamond electrodes are hydrophobic, which leads to undesirable protein denaturation and adsorption of concomitant non-specific biomolecules.

Moreover, the publication "Electrochemical analysis of nucleic acids at boron-doped diamond electrodes" (Prado, C. et al., Analyst 127, 329-332 (2002)) describes a method of detecting of nucleic acids by a BDD electrode by using cyclic voltammetry (CV) technique and square wave voltammetry.

Another publication "Development of Amperometric ImmunosensorUsing Boron-Doped Diamond with poly(o-aminobenzoic acid" (Anchana Preechaworapun et al. Anal. Chem. 2008, 80, 2077 - 2083) describes alternative method of preparing a protein immunosensor. In order to construct the base of the immunosensor, o-aminobenzoic acid (o-ABA) was electropolymerized at an electrode by cyclic voltammetry. The XPS result found that carboxyl groups were formed at the electrode surface. The carboxyl groups were then used to covalently attach protein probes. Such kind of surface modification has been shown in the publication "Doping Level of Boron-Doped Diamond Electrodes Controls the Grafting Density of Functional Groups for DNA Assays" (Da-Jung Chung et al. Electrochimica Acta 76 (2012) 394-403) as well. The authors have have demonstrated that 4-carboxy phenyl groups based modification could be performed on different electrodes, such as indium tin oxide (ITO), gold (Au), and glassy carbon electrode (GCE). The electrode modification has been performed through cyclic voltammetry by sweeping the potential between +1.0 V and -1.0 V for 'n' number of potential cycles. Subsequently, the electrochemical DNA sensor (E-DNA sensor) based on the 4-carboxy phenyl modified GCE was fabricated by the immobilization of probe DNA.

Borond-doped diamnods were also described in "Electrically addressable deposition of diazonium- functionalized antibodies on boron-doped diamond microcells for the detection of Ochratoxin A" (Amani Chrouda et al., Anal. Methods, 2015,7, 2444-2451) - manufacturing procedure for an impedimetric immunosensor for sensitive detection of the mycotoxin, ochratoxin A (OTA), through electroaddressing of diazonium functionalized antibodies on the working electrode of a planar Boron Doped Diamond (BDD) electrochemical microcell and in "Doping Level of Boron-Doped Diamond Electrodes Controls the Grafting Density of Functional Groups for DNA Assays (Lubomir Švorc et al., Appl. Mater. Interfaces 2015, 7, 18949-1895) where BDD has been grafted by electrochemical reduction of aryldiazonium salts. The grafting e ffi ciency of 4-nitrophenyl groups increased with the boron levels (B/C ratio from 0 to20 000 ppm). Controlled grafting of nitrophenyldiazonium was used to adjust the amount of immobilized single-stranded DNA strands at the surface and further on the hybridization yield in dependence on the boron doping level.

In "Label free biosensor for sensitive human influenza virus hemagglutinin specific antibody detection using coiled-coil peptide modified microelectrode array based platform" (Sunil K. Arya et al., Sensors and Actuators B 194 (2014) 127-133) coiled-coil peptide (CCP) modified microelectrode array with comb structure (MACS) has been utilized to fabricate an electrochemical biosensor for antibody detection. Thiol terminated CCP has been employed for self-assembling on MACS and peptide chains connecting amino acid sequence 98-106 (YPYDVPDYA) of human Influenza virus hemagglutinin (HA) has been used as a molecular receptor for HA-antibody.

Besides the BDD substrate, there are also known other materials (e.g. Ag, or alumina and tantalum oxides) which can serve, upon suitable surface modification, as working electrodes in various electrochemical biosensors. Regardless the chemical composition, the process of a substrate surface modification involves immobilization of certain probe biomolecules on the surface of the substrate. Depending on the substrate affinity, there are two different ways to immobilize the probe biomolecules on the substrate.

One method is the direct attachment method, wherein the probe biomolecules attach to the substrate surface due to interactions between the probe molecule and substrate surface. However, direct attachment may lead to low surface coverage, which is especially important in detection of proteins and antibodies of low sample concentration.

Another method for immobilizing constitutes indirect immobilization of the probe biomolecules via certain linker molecules. The linker molecules attach directly to the surface of the substrate forming an organic monolayer facilitating further attachment of the probe biomolecules. Therefore, in this method the probe biomolecules are attached via linker molecules forming a monolayer on the suitable detecting surface.

The selection of appropriate linker molecules to form an organic monolayer of desirable properties plays an important role, especially in EIS (Electrochemical Impedance Spectrometry) sensors that employ substrates with immobilized probe biomolecules as working electrodes. The requirements that must be met by the organic monolayer of linker molecules in an EIS sensor are: high surface coverage, sufficient blocking of the active surface in order to prevent non-specific adsorption of interfering molecules and appropriate length of the formed organic layer. The aforementioned aspects have considerable impact on EIS sensors sensitivity, stability and packaging density of the working electrode. The known linker molecules, which are suitable for forming organic linker layers on the substrate serving as working electrodes in EIS sensors, are alkanethiols: 11-MUA, thioctic acid and 3-MPA.

A method of biosensing using EIS sensors comprises estimation of the concentration of an analyte (a molecule of interest which is being analyzed) in a sample solution. The selected probe biomolecules should exhibit compatibility with the analyte biomolecules. To perform the EIS detection assay, the substrate with immobilized probe biomolecules is immersed with a sample solution containing the analyte. The analyte diffuses through the solution. These analyte biomolecules which reach surface of the substrate, bind to the probes at the capture phase. Subsequently, the concentration of the analyte-probe complexes, immobilized on the electrode surface, is measured by using the EIS detection method, which performs the transduction of the signal from the psychochemical domain to a electrochemical domain.

Depending on the selected probe biomolecules, the EIS sensors may be for example used for detection of biomolecules produced by certain pathogens, and thus, the EIS sensor may serve as a diagnostic apparatus for detection of various diseases such as viral or bacterial infections.

There are known various EIS sensors for detection of various pathogens in biological samples.

A publication "Universal biosensor for detection of influenza virus" (D. Nidzworski et al., Biosensors and Bioelectronics, Volume 59, 15 September 2014, pages 239-242) discloses a sensor for detection of influenza virus in swabs taken from the human throat. The biosensor is an impedance electrochemical spectroscopy meter in which a modified gold electrode serves as a working electrode. The electrode surface is modified by direct attachment of the M1 antibodies of the influenza virus to the electrode surface. The sensor can detect the influenza virus in a sample with concentration of the virus of 60 pg/ml.

A PCT patent application WO2014014371 discloses a method for preparing an immunosensor for detection of influenza virus which involves rinsing gold electrode with ethanol and immersing the electrode in an ethanol solution of 1,6-hexanedithiol for around 20 hours, at room temperature, rinsing the electrode with ethanol and deionized water, and turning the electrode upside down. Subsequently, a gold solution (GPC) is applied on the electrode. Then, the electrode should be stored for 18 hours in 4 °C. Next, the electrode is rinsed with PBS, and anti-M antibodies of the influenza virus are applied on the electrode. The prepared electrode is ready to use and serves as an immunosensor for detection of the influenza virus.

As follows from the above referred publications, there are known various methods for modification of various substrates to be used as working electrodes in EIS sensors for detection of biological molecules in sample solutions. These methods are developed with the aim to provide substrates that feature good surface adsorption of various biological molecules, as well as to provide an EIS sensor that will show improved sensitivity, selectivity, stability and shorter detection time.

Therefore, there exists a need for further development of the substrate that can be suitable for implementation, as a working electrode, into an EIS sensor, providing an electrode of an EIS sensor of improved stability, sensibility, and selectivity that is suitable for detection of various biological molecules (analytes), such as amino acids, peptides, proteins, hormones, sugars or nucleic acids.

Moreover, the currently known systems for monitoring pathogens have a disadvantage related to performing measurements and analysis for each patient individually. However, there are cases where global or selective analysis of chosen population individuals is recommended. Therefore, there is a need to design and implement an effective method and a system for monitoring pathogens, which monitors spreading of infections and predicts the progress of a development of an epidemic or a pandemic.

SUMMARY

The invention is defined in the claims. There is disclosed herein a modified BDD (boron doped diamond) substrate comprising: an organic layer comprising carboxyl groups, where the organic layer on the BDD substrate comprises organic groups perpendicularly attached to the surface of the substrate, wherein organic groups have been electrochemically immobilized from diazonium salt of 4-aminobenzoic acid in an AC scanning CV (Cyclic Voltammetry) process, and the substrate further comprises probe molecules covalently bound to the carboxyl groups of the organic layer.

The substrate where the AC scanning signal has a negative potential of up to-1 V and a scanning rate of 50 to 200 mV/s wherein the organic layer is formed in the CV electrochemical cell comprising a silver-chloride (Ag/AgCl) electrode as a reference electrode and a platinum electrode as a counter electrode and/ or the organic layer is formed by applying the scanning signal in a plurality of cycles, preferably 10 cycles.

The modified BDD substrate may further comprises analyte molecules bound to probe molecules wherein the modified BDD substrate comprises, as the analyte molecules, at least one molecule selected from the group consisting of: amino acids, hormones, sugars, nucleic acids, peptides and proteins and/ or the modified BDD substrate comprises, as the probe molecules, anti-M1 proteins of influenza virus or the modified BDD substrate comprises, as the analyte molecules, M1 proteins of influenza virus

There is also disclosed an impedance sensor for detecting biological molecules, comprising an EIS (Electrochemical Impedance Spectroscopy) meter, wherein the sensor comprises, as a working electrode, the modified BDD substrate as described above.

The sensor may comprise as an electrolyte, a solution of 1 mM $K_3Fe(CN)_6$ / 0,1 PBS and as a reference electrode, a chloro-silver electrode: Ag/AgCl/0,1 M KCl and as a counter electrode, a platinum (Pt) electrode.

There is also disclosed a method for detecting biological molecules, the method comprising the steps of: providing the sensor as described above; incubating the working electrode of the sensor with a diluted biological sample; immersing the working electrode of the sensor in an electrolyte of the EIS cell; conducting an EIS measurement at an AC current to register EIS measurement results; and comparing the EIS measurement results with reference EIS data obtained for a sample without analyte biomolecules.

The method may comprise using an anti-M1 protein of an influenza virus as the probe biomolecule or an analyte biomolcule.

BRIEF DESCRIPTION OF THE DRAWINGS

The present disclosure is presented by way of examples on a drawing, in which:

Fig. 1 presents schematically an exemplary method for preparing a modified BDD electrode;
Fig. 2 presents schematically a an exemplary method for preparing an organic layer on a surface of a BDD electrode;
Fig. 3 presents schematically, in greater detail, a an exemplary method for preparing a modified BDD electrode;
Fig. 4 presents Cyclic Voltammetry (CV) curves registered for a non-modified and for a modified BDD electrode, immersed in 1 mM $K_3Fe(CN)6$ + 0,1 PBS, recorded with a scan rate of 50 mV/s;
Fig. 5 presents a table with collected values of elements calculated from the EEQC for a non-modified and for a modified BDD electrode incubated in different solutions;
Fig. 6 presents impedance spectra of a non-modified and of a modified BDD electrode after incubation in different solutions, immersed in 1 mM $K_3Fe(CN)_6$ + 0,1 PBS at $E_f$ = + 0,13 V vs. Ag/AgCl/0,1 M KCl;
Fig. 7 presents a table with collected values of elements calculated from the EEQC for a modified BDD electrode incubated in different solutions of the M1 protein;
Fig. 8 presents a bar graph with values of charge transfer resistance, for two electrodes incubated separately with a W3 and with a W5 virus sample; EIS measurement taken in 1 mM $K_3Fe(CN)_6$ in 0,1 PBS;
Fig. 9 presents a table with collected values of elements calculated from the EEQC for a modified BDD electrode incubated in W3 and W5 virus sample diluted in 0,5% Triton X-100/0,1 PBS solution;

Fig. 10 presents Cyclic Voltammograms (CV) of BDD electrode in contact with diazonium salt of 4-aminobenzoic acid in diluted HCl (ten cycles performed at a scan rate of 100 mV/s).

## DETAILED DESCRIPTION

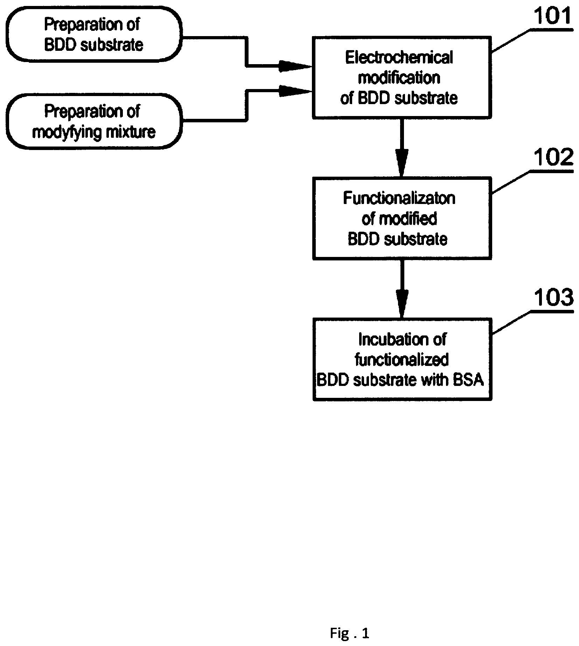

Figs. 1-10 relate to a modified BDD substrate, a method for preparing thereof and an ultrasensitive EIS sensor, for detection of various biological molecules.

## PREPARATION OF A MODIFIED BDD SUBSTRATE

Fig. 1 presents schematically a method for modifying a surface of a boron doped diamond (BDD) substrate according to the present disclosure. The method comprises a step of electrochemical modification of the BDD surface with a modifying mixture based on diazonium salt of aminobenzoic acid followed by immobilization of probe biomolecules on the modified BDD substrate.

In order to prepare a modifying mixture for use for the modification of the BDD surface, aminobenzoic acid is dissolved in a concentrated sulfuric (VI) acid, followed by introduction sodium nitrite ($NaNO_2$). A water mixture is obtained comprising: aminobenzoic acid in concentration of 0,26 to 0,30%, sulfuric (VI) acid in concentration of 28 to 30% and sodium nitrite in concentration of 0,30 to 0,36%.

The obtained mixture is subsequently mixed, e.g. with a magnetic stirrer, at a temperature of about 4°C, for 15 - 40 minutes. The result of the mixing process is the modifying mixture that is a solution of diazonium salt of aminobenzoic acid. The prepared mixture is subsequently used for electrochemical modification of the surface of the BDD substrate.

The BDD substrate, that is modified in step 101, may be prepared using various techniques known in the art. For example, the BDD substrate may be prepared in a process that involves: depositing a nanodiamond suspension onto a Si substrate and doping the nanodiamond layer with boron. The use of the substrate that contains only a thin surface layer of BDD provides reduction of costs of the BDD substrate. Nonetheless, a substrate made entirely of BDD can be used as well in the presented method.

Following the preparation, the BDD substrate should be modified in the process of electrochemical deposition of an organic layer onto the surface of the BDD substrate 101. To this end, the technique of Cyclic Voltammetry (CV) is used, wherein the BDD material is used as the working electrode of a CV cell, a silver-chloride (Ag/AgCl) electrode is used as a reference electrode, and a Pt wire is used as a counter electrode. The modification process of the BDD material comprises immersing the electrodes: BDD, Ag/AgCl and Pt in a prepared modifying mixture based on diazonium salt of aminobenzoic acid. The modification (electrochemical deposition) process is conducted analogously to the CV measurement, i.e. by applying to the working electrode an AC scanning signal within the range of 0 V to -1 V, at a scanning rate of 50-200 mV/s, preferably 100 mV/s, preferably in 10 successive cycles. The parameters are optimized within these ranges in order to avoid dissociation of water, which could cause generation of hydrogen, oxygen and hydroxyl radicals, which would damage the organic matter. Negative potential is necessary to cause modification and polymerization of chain fragments that have a positive potential. Subsequently, the modified BDD substrate is removed from the modifying mixture and it is rinsed with alcohol, in order to remove impurities which are not covalently bonded with the BDD substrate. The modified BDD substrate obtained this way may be dried with an air stream or it may be left to dry.

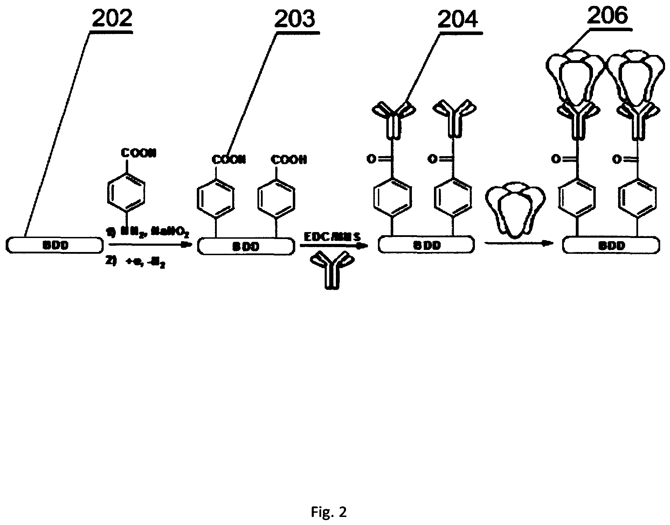

As shown in Figs. 2 - 3, at steps 202 - 203, during modification process, the AC scanning signal is applied to the BDD substrate, which is employed in the CV cell as the working electrode which is immersed in the modifying mixture. This provides the reduction of diazonium salt of aminobenzoic acid, which further leads to generation of aryl-free radicals which attack the surface of the BDD substrate and form stable, covalent bonds with the BDD material, thereby forming the organic layer which is covalently bonded to the BDD substrate.

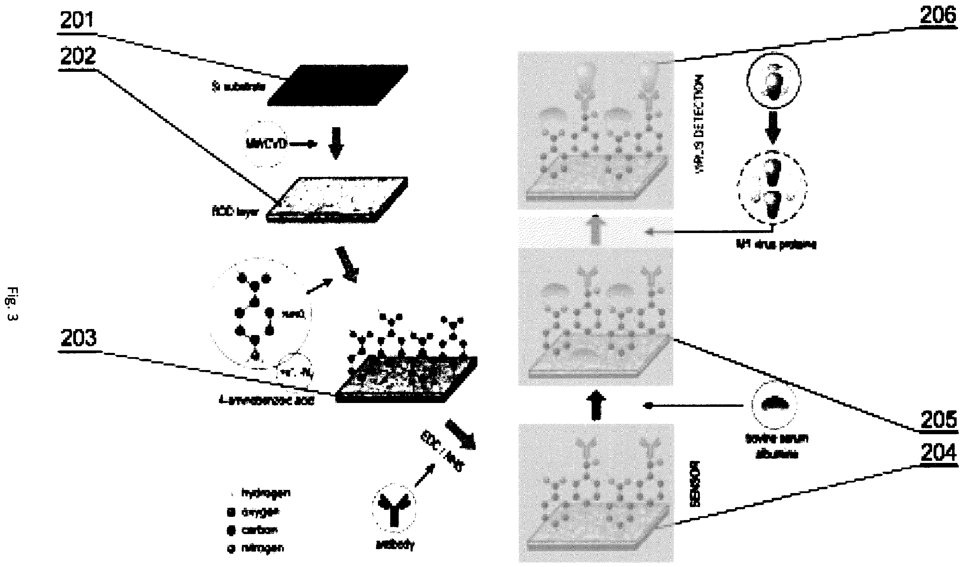

The modified BDD substrate obtained this way (Figs. 2, 3) has a surface immobilized organic layer 203 that contains aryl moieties, wherein each aromatic ring of the aromatic moiety has a carboxylic group (-COOH) as the substituent, and wherein the position of the substituent can be: ortho-, meta- or para-, with respect to the covalent bond of the aromatic ring to the BDD substrate. Therefore, the organic layer (the linker layer) covalently bonded to the BDD substrate comprises free carboxylic groups 203 that can be subjected to further modification.

The position of carboxyl substituents (ortho-, meta- or para-) in the aromatic ring depends on the molecular structure of diazonium salt of aminobenzoic acid present in the modifying mixture. As shown in Figs. 2 and 3, when using diazonium salt of 4-aminobenzoic acid, the organic layer is obtained having carboxylic groups in the para-substitution - with respect to the position of the covalent bond: aromatic ring-BDD substrate 203.

Depending on special needs, e.g. molecular structure of the probes to be immobilized on the organic layer, there may be used modifying mixtures comprising: diazonium salt of 4-aminobenzoic acid, diazonium salt of 2-aminobenzoic acid or diazonium salt of 3-aminobenzoic acid or mixture thereof, providing respective substitution (otrho-, meta- or para-) of the carboxyl group in the aromatic rings of the organic layer.

The carboxyl groups of the organic layer immobilized on the BDD substrate 203 can be subsequently functionalized with various biological molecules (probe), at step 103 (Fig. 1). The functionalization process involves formation of a stable, covalent bond, such as ester or amide bond, between the probe biomolecule and carboxyl groups of organic layer, as schematically shown in Figs. 2 and 3 (203 - 204).

Therefore, the organic layer immobilized on the BDD surface 203 serves as a linker favoring immobilization of certain biological molecules (probes) on the BDD substrate. The probe biomolecules that may be immobilized on the modified BDD substrate are, for example: amino acids, peptides, proteins, hormones, sugars or DNA fragments. The reaction involving formation of a covalent bond of various probe biomolecules to the modified BDD substrate is schematically shown in Fig. 2 and Fig. 3, and Fig. 4 schematically presents an example of an embodiment of the reaction in which, an antigen: anti-M1 protein of influenza virus (serving as a probe) binds to the organic layer of the modified BDD substrate 203 - 204.

The immobilization of the probe biomolecules on the modified BDD substrate 203 - 204 may be accomplished, for instance, by incubation of the surface of modified BDD substrate in a respective solution of probe biomolecules.

For example, the reaction in which the antigen anti-M1, constituting probe biomolecule, bonds to the modified BDD substrate 203 - 204, may be accomplished as described below.

The modified BDD substrate may by incubated in the solution that comprises: a probe: antigen anti-M1 and the mixture of N-Ethyl-N'-(3-dimethylaminopropyl)carbodiimide hydrochloride(EDAC) / N-hydroxysuccinimide. The functionalization process is preferably conducted in the presence of dimethyl sulfoxide (DMSO) with addition of phosphate buffer of pH 5,5, at the temperature of 4°C, for 24 hours.

The anti-M1 protein may be also anchored to the modified BDD substrate in the process of incubation of the modified surface of the BDD substrate with water or phosphate buffer solution of anti-M1 protein or in the process of incubation of the modified surface of the BDD substrate with the mixture of dimethylformamide (DMF) and methylene chloride mixed in the volume ratio of 1:1.

Exemplary, not being the part of the invention, after the functionalization process, the BDD substrate may be treated with bovine serum albumin (BSA). The treatment provides nonspecific binding of the BSA to the organic layer of the BDD substrate, as schematically shown in Fig. 3: 204 - 205. The treatment with BSA may be accomplished, for example, by incubation of the functionalized BDD substrate with 0,1 M BSA water solution, at the temperature of 4°C, for 1 hour.

CONSTRUCTION OF EIS SENSOR COMPRISING A MODIFIED BDD SUBSTRATE

The modified BDD substrate, prepared according to Fig. 1 and Figs 2-3: at steps: 201 - 205, with immobilized organic layer and anchored, on its surface, respective probe biomolecules, may serve as a working electrode of an EIS (Electrochemical Impedance Spectroscopy) sensor. The EIS sensor with the modified BDD electrode according to the present disclosure features high specificity, universality and sensitivity, and it is capable of detecting various biological molecules in a sample solution providing, at the same time, short detection time and a non-complicated detection procedure.

The EIS sensor with the modified BDD electrode may be in a form of a conventional Electrochemical Impedance Spectroscopy (EIS) meter for impedance evaluation comprising an electrochemical cell with electrodes immersible in an electrolyte, a potentiostat / a galvanostat and a frequency response analyzer (FRA) which applies a sine wave and analyses a response of the system to determine the impedance.

The electrochemical cell of the EIS sensor, according to the present disclosure, comprises (as a working electrode) a modified boron-doped diamond (BDD) substrate comprising an organic layer and, immobilized on the organic layer, the probe biomolecules that are suitable for binding with respective analyte biomolecules when present in the analyzed sample solution. The probe biomolecules that may be immobilized on the BDD modified substrate are for example polyclonal anti-M1 antibodies, in case when the EIS sensor is used for detection of the M1 protein of influenza virus. Nonetheless, amino acids, peptides, proteins, hormones or DNA fragments may be also used as probe biomolecules, depending on the type of analyte biomolecule being investigated.

The electrochemical cell of the EIS sensor may be of another known electrode configuration, and preferably comprises three electrodes: a working electrode which is the BDD substrate modified with suitable probe biomolecules, a counter electrode to close the electrical circuit, such as for example platinum (Pt) electrode, and a suitable reference electrode to determine the potential across the electrochemical interface accurately, such as for example Ag/AgCl/0,1 M KCl electrode.

STUDIES AND ELECTROCHEMICAL CHARACTERIZATION OF MODIFIED BDD SUBSTRATE

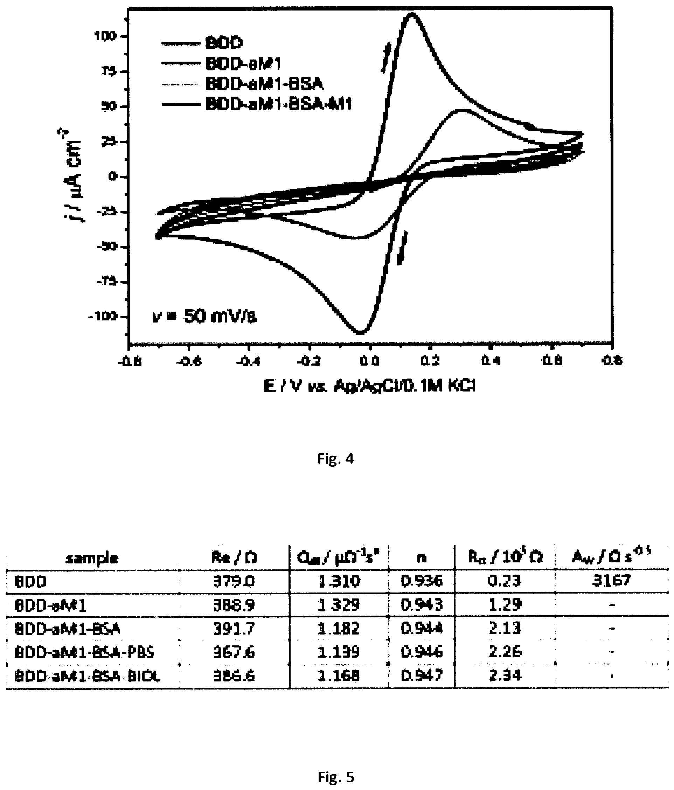

In order to evaluate the properties of the modified BDD substrate, CV studies were conducted on various steps of its preparation on reversible behavior of the BDD substrate, employing the BDD substrate as a working electrode of a CV cell, towards $Fe^{2+}/Fe^{3+}$ redox active species. The results of the CV studies have shown a remarkable change in oxidation-reduction properties of the BDD substrate during its preparation. The Cyclic Voltammetry (CV) curves registered for a non-modified (pristine) BDD electrode and a BDD electrode modified with the anti-M1 protein immersed in 1 mM $K_3Fe(CN)_6$ + 0,1 PBS, with the scan rate of 50 mV/s are shown in Fig. 4.

As follows from the CV curves of Fig. 4, for the non-modified (pristine) BDD electrode (denoted as BDD), one may observe the usual set of reduction and oxidation current related to a change in oxidation state of iron, where the difference between the oxidation and the reduction potential peak equals $\Delta E = 0,17$ V. For the modified BDD electrode with the immobilized organic layer and probe biomolecules: anti-M1 protein (denoted as BDD-aM1), the electrode response is still reversible but the peak current decreases while $\Delta E$ increases and equals $\Delta E = 0,36$ V, respectively. Although for the modified BDD electrode incubated with BSA (denoted as BDD-aM1-BSA) any oxidation or reduction peak in not observed, and the registered CV curve (Fig. 4) is characterized only by a small change in current. Such a drastic change in CV character results from efficient blockage of electron transfer between electrode and redox active species present in the electrolyte solution of 1 mM $K_3Fe(CN)_6$ + 0,1 M PBS.

Moreover, the same CV studies were performed on the BDD, for exemplary purposes only,electrode with an anchored analyte: M1 protein (denoted as BDD-aM1-BSA-M1), not being the part of the invention. In order to prepare the BDD-aM1-BSA-M1 electrode, a BDD-aM1-BSA electrode was incubated with a M1 protein solution (50 fg/mL). The incubation process was schematically shown in Fig. 3: 205 - 206. The resulting CV curves of BDD-aM1-BSA-M1 showed no significant change in the registered CV curve. Thus, the CV curves allow to follow the change upon a probe, such as for example anti-M1-protein modification, and BSA unspecific binding, nonetheless CV measurement cannot be regarded as a proper diagnostic tool for confirmation of formation probe-analyte complexes on the surface of modified BDD electrode.

In the course of further research (Fig. 5), different modified BDD substrates (samples) were employed into the EIS cell - each substrate was serving as a working electrode. The electrochemical impedance spectra were recorded for the following BDD substrates:

- a non-modified (pristine) BDD electrode (denoted as BDD);
- a modified BDD electrode with an immobilized organic layer modified with probe biomolecules: anti-M1 proteins (denoted as BDD-aM1); and
- a modified exemplary, not being the part of the invention, BDD electrode with an immobilized organic layer modified with the anti-M1 protein and treated with BSA (denoted as BDD-aM1-BSA), respectively.

Additionally, two more EIS spectra were recorded for:

- a modified BDD exemplary, not being the part of the invention, electrode (BDD-aM1-BSA) further incubated with 0,1 M PBS (denoted as BDD-aM1-PBS); and
- a modified BDD electrode further incubated with a diluted biological sample taken from a swab of a healthy patient (denoted as BDD-aM1-BIOL).

All electrochemical impedance spectra were recorded in 1 mM $K_3Fe(CN)_6$ + 0,1 PBS at a formal potential of $Fe^{2+}/Fe^{3+}$ redox reaction of $E_f = 0,13$ V with respect to a reference electrode: Ag/AgCl/0,1 M KCl.

The shape of spectrum which was recorded for the pristine BDD electrode was analyzed using an electric equivalent circuit Re [CPE($R_{ct}$W)] consisting of an electrolyte resistance $R_e$ connected in series with a parallel connection of a constant phase element (CPE) and a series connection of a charge transfer resistance $R_{ct}$ and a Warburg element W assigned to the diffusional resistance. However, in the case of a modified BDD electrode, on the impedance spectra, the characteristic 45-degree line was not observed, thus the Warburg element was omitted.

The values of each element of the electric equivalent circuit (EEQC) are shown in the table in Fig. 5. The fitting procedure provides normalized fitting errors in optimal solution on the level of 10-4. The constant phase element can be viewed as a heuristic method, in order to incorporate the effect of surface heterogeneity along and through the electrode/electrolyte interface. The impedance of the constant phase element (CPE) is characterized by impedance Z = Q-1(i$\omega$)-n, where: $\omega$ is angular frequency, Q is the CPE parameter, i is the sinusoidal component and n is the factor of imperfection of the element. The n values for the CPE elements are c.a. 0,95.

The Ohmic resistance element in the high frequency regime attributed to the electrolyte resistance is similar for all tested materials and fits the range of 360-380 Ohms. Comparing the spectra registered for non-modified (pristine) BDD electrode with the spectra registered for the BDD electrode modified with biomolecules (anti-M1 protein) and with the spectra recorded for modified BDD electrode incubated with BSA solution, one may observe significant increase in resistance ($R_{ct}$) value. The increase in $R_{ct}$ value suggests successful binding of the probe biomolecules (antigen: anti-M1) to the surface of the BDD electrode functionalized with diazonium salt of aminobenzoic acid, where incubation with BSA provides formation of unspecific binding of BSA to the organic layer of the BDD surface.

In an example, not being part of the invention, the anchored probe (M1 antibody) and BSA linked to the organic layer of the BDD electrode efficiently hamper the charge transfer process, resulting in a remarkable increase of $R_{ct}$ values. Moreover, the electrochemical response of the BDD-aM1-BSA electrode upon the presence of 0,1 PBS or biological sample is not affected. The change in charge transfer resistance was less than 10% and it was repeatable for all five tested BDD-aM1-BSA electrodes - as results from comparing $R_{ct}$ values with each of the tested electrode before and after incubation with either 0,1 M BSA or biological sample.

As follows from the above experiment, the modified BDD substrate is suitable for serving as a working electrode of the impedance (EIS) sensor for determination the presence of the various analyte biomolecules, such as for example the M1 protein of the influenza virus.

METHOD FOR DETECTING BIOLOGICAL MOLECULES USING AN EIS SENSOR

A method for detecting a respective analyte in a solution, by using the impedance (EIS) sensor comprising the modified BDD substrate as the working electrode, comprises the following steps:

- providing the EIS-sensor comprising the BDD modified substrate as the working electrode (the modified BDD substrate being treated with diazonium salt of aminobenzoic acid in the CV experiment, exemplary subsequently incubated with BSA, and incubated with probe solution (Fig. 2, Fig. 3 elements: 201 - 205);
- incubating the working electrode with the solution of analyte (e.g. the biological sample);
- contacting the working electrode with the electrolyte of the EIS cell;
- conducting the electrochemical impedance spectroscopy measurement (employing the modified BDD substrate as a working electrode);
- optionally, rinsing the electrode with proper substance in order to remain on the electrode surface only permanently bonded analytes (analyte-probe complexes); the rinsing substance may be for example 0,1 M solution of phosphate-buffered saline (PBS); and
- analyzing the obtained EIS spectrum, involving comparison of the spectra obtained for investigated analyte with an EIS reference spectrum - the spectrum recorded for the BDD modified electrode incubated with a blank sample (i.e. the sample of the same composition, but not comprising the analyte biomolecules).

The EIS measurement for the blank sample and for the investigated biological sample (analyte) may be carried out, for example, by using the same DBB electrode and in the same conditions - to minimize the influence of changes in external factors (temperature, humidity) on the assay results. Preferably, the modified BDD electrode (BDD - organic layer - probe) should be incubated in the first step with the blank sample followed by the registration of the reference EIS spectrum, and in the next step the same electrode, after suitable rinsing, should incubated with the investigated sample (i.e. the sample comprising analyte biomolecules) followed by registration of EIS spectrum. Nonetheless, the reference spectra or other reference values calculated on the basis of the obtained EIS reference data - for different blank samples, analyzed in different conditions, may be provided in the form of reference data sheet and used in the assay as reference values. In such a case, the step of recording the EIS reference spectra may be omitted, and the values recorded (by using EIS technique) for the investigated sample may be compared with the values of reference data sheet, thereby, shortening the time of the analysis.

The method for detecting analyte biomolecules may additionally involve, as an initial step, collecting an analyte sample, which may be taken from a living organism, e.g. from a human or an animal body, such as for example: swab, urine, blood or another tissue.

For instance, in case of detection of influenza virus, the initial detection step may involve collection of swab from a patient's throat, which may be subsequently transferred to a suitable substance that allows to release the M1 protein out of the viral cell. As a suitable substance, a mixture of 0,5% Triton X-100/0,1 PBS may be used, because it does not affect the response of the BDD modified electrode in EIS cell, and therefore does not influence obtained result of the measurement.

Preferably, the EIS measurement is carried out in a solution of 1 mM $K_3Fe(CN)_6$ + 0,1 PBS. Nonetheless, other suitable electrolytes with Fe2+ or Fe3+ ions may also be used.

Preferably, platinum (Pt) wire is used as a counter electrode, but other suitable metals may also be used instead, for example: gold (Au) or silver (Ag)

Preferably, Ag/AgCl/0,1 M KCl is used as a reference electrode, but other suitable substances may be used as the reference electrode, for example: Ag/AgCl/0,1 M NaCl or calomel Hg /$Hg_2Cl_{2(s)}$ /$KCl_{(nas.)}$.

Preferably the EIS measurements are conducted at a frequency range from 20 kHz to 0,1 Hz, at a small amplitude alternating current (preferably about 10 mV) and at a formal potential of the redox reaction: $F^{2+}/Fe^{3+}$ of $E_f =$ 0,13 V.

AN EXAMPLE OF A METHOD FOR ANALYZING OBTAINED EIS SPECTRA

After registration, the obtained assay results (EIS spectra) may be analyzed on the basis of an electric equivalent circuit (EEQC), for example by using an EIS Spectrum Analyzer program, which uses the modified Powell algorithm to calculate amplitude weighting $r_a$ coefficient according to the formula I:

$$r_a(\omega, P_1...P_M) = r_c^2 /(N-M) \quad \text{formula I}$$

wherein N is the number of points, M is the number of parameters, $\omega$ is the angular frequency, P1...PM are parameters. The parameter $r_c$ is defined as:

$$r_c^2 = \sum_{i=1}^{N} \frac{\left(Z_i' - Z_{i_{calc}}'\right)^2 + \left(Z_i'' - Z_{i_{calc}}''\right)^2}{Z_i'^2 + Z_{i_{calc}}'^2} \quad \text{formula II}$$

wherein i corresponds to the measured values of impedance and $i_{calc}$ is attributed to the calculated values; N is the number of points.

DETERMINATION OF THE DETECTION LIMIT OF THE EIS SENSOR

In order to verify the detection limit of the EIS sensor, according to the present disclosure, the anti-M1 protein was immobilized on the modified BDD substrate (with the organic layer) as a probe biomolecule, and the obtained substrate (denoted as BDD-aM1-BSA) was subject to testing with dilute solutions of different concentrations of corresponding analyte biomolecule: M1 protein of influenza virus, in order to obtain probe-analyte complexes on the modified BDD substrate. The modified BDD substrate was subsequently implemented as a working electrode of the EIS cell, in which the electrode response with respect to each solution of the M1 protein was measured by using electrochemical impedance spectroscopy. The modified, not being part of the invention, BDD electrode (BDD-aM1-BSA) was prepared in advance according to the method described herein and incubated with each solution of the M1 protein. The test involved evaluation of the solutions of the M1 protein of the following concentrations: 10 fg/mL, 50 fg/mL, 100 fg/mL and 8 pg/mL, respectively. For each concentration of the M1 protein, the electrode (BDD-aM1-BSA) was incubated with a respective solution, and then rinsed with fresh 0,1 M PBS. Subsequently, the surface of the electrode was immersed in the electrolyte of 1 mM $K_3Fe(CN)_6$ + 0,1 PBS, Pt wire was used as a counter electrode and Ag/AgCl/0,1 M KCl as a reference electrode. When the electrode temperature had reached equilibrium with external conditions, the electrochemical measurements were initiated. After each electrochemical measurement, the electrode surface was washed once again with a fresh solution of 0,1 M PBS.

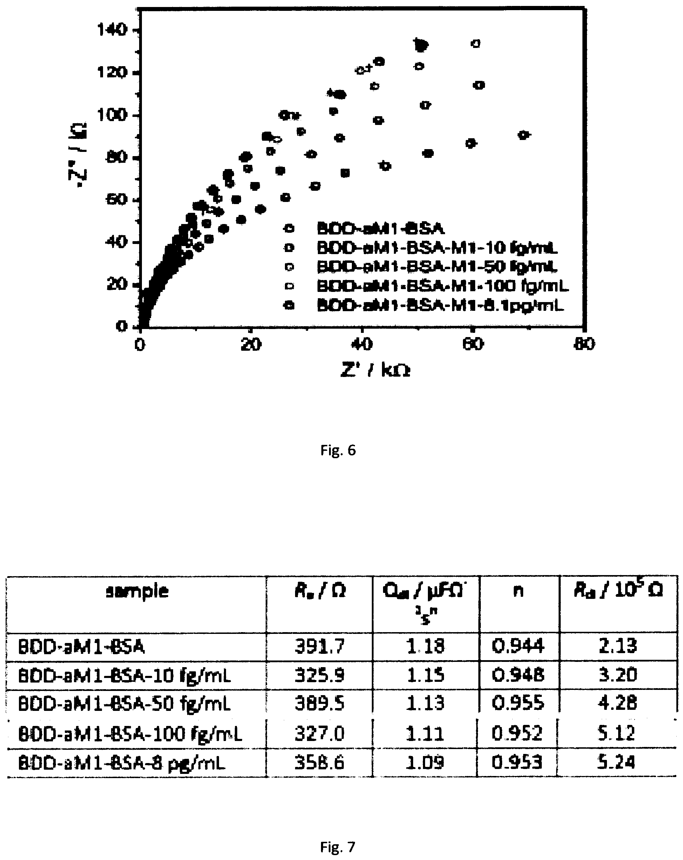

Figs. 6 and 7 present the results of the experiment, wherein: Figs. 6 presents the impedance spectra registered after each incubation - in each M1 protein solution which have been recorded in 1 mM $K_3Fe(CN)_6$ + 0,1 PBS at $E_f$ = 0,13 V; and Fig. 7 presents a table containing the values of elements calculated from the EEQC for the modified BDD electrode (for example, being beyond the scope of the invention, BDD-aM1-BSA) incubated with the solution of the M1 protein of a different concentration. Because after each incubation, the electrode surface was washed with a fresh 0,1 PBS solution, only the permanently bonded proteins (probe-analyte complexes) remain on the BDD surface.

As follows from Figs. 6, 7, after incubation with the samples of 10 fg/mL, 50 fg/mL and 100 fg/mL of the total concentration of the M1 protein, a significant change in the shape of the EIS spectra is observed. According to the resistance values collected in the table of Fig. 7, upon subsequent incubation, the $R_{ct}$ value of electrode changed by 50%, 101% and 140%, for the electrode (BDD-aM1-BSA) being in contact with sample of concentration of the M1 protein of 10 fg/mL, 50 fg/mL and 100 fg/mL, respectively, assuming that the resistance ($R_{ct}$) of the electrode with no M1 protein bonded to the surface equals 0%. This visible change is related to successive formation of probe-analyte complexes (anti-M1 protein/M1 protein), which leads to formation of a dense cover on the BDD electrode (substrate). This blocks efficient transfer of electrons between the electrode and the redox active species ($Fe^{2+}/Fe^{3+}$) present in electrolyte (1 mM $K_3Fe(CN)_6$ + 0,1 PBS) which is seen as an increase in $R_{ct}$ value. After incubation of the electrode with a solution of a higher concentration of the M1-protein, i.e. 8 pg/ml, the impedance spectrum changes slightly, which is in the range of measurement error. Therefore, incubation of the electrode in the solution of the M1 protein of a higher concentration does not cause a visible change the resistance, due to the lack of the active sites (i.e. free anti-M1 protein attached to the organic layer of the BDD substrate). Therefore, incubation with 100 fg/mL concentrated solution (40 $\mu$L) of the M1 protein leads to complete saturation of all active sites (probes bonded to organic layer of the BDD substrate).

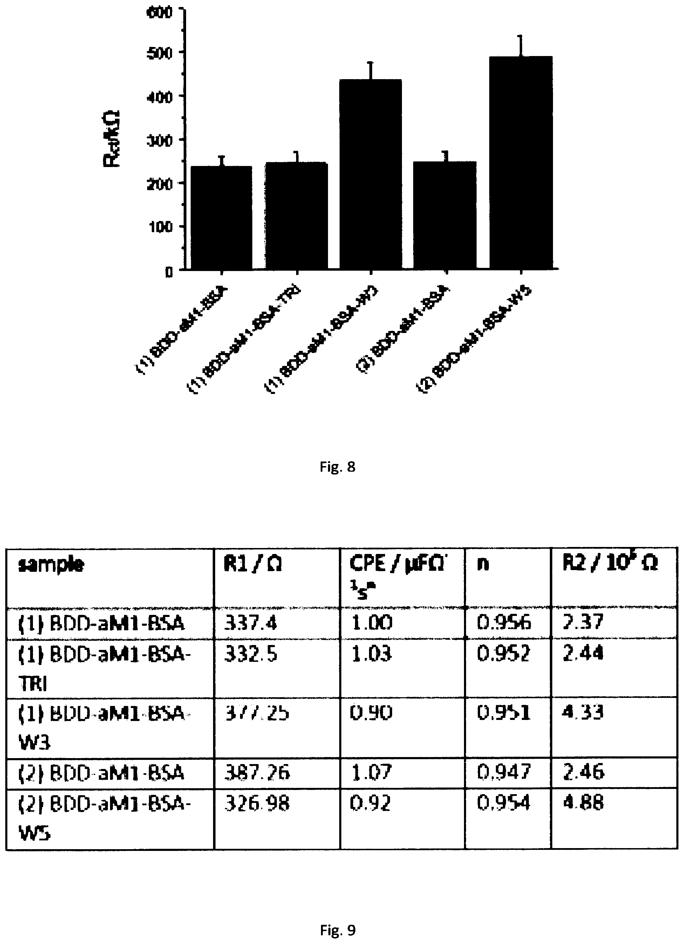

In order to evaluate the detection capabilities of the sensor, according to the present disclosure, a further experimental procedure has been conducted, in which the sensor, comprising the BDD modified electrode with the immobilized anti-M1 protein, was exposed to M1 proteins that were released from real viruses samples: W3 (avian influenza A (H5N2)) and W5 (human influenza A (pandemic H1N1 strains)). The samples were prepared in a mixture of 0,5% Triton X-100/0,1 M PBS. The tests were performed by using an EIS sensor in which the modified BDD substrate was used as a working electrode, Pt electrode was used as a counter electrode and Ag/AgCl/0,1 M KCl was used as a reference electrode. In the assay, two BDD exemplary modified electrodes were used: (1) a BDD-aM1-BSA electrode for measurement of charge transfer resistance ($R_{ct}$) of the sample with M1 protein of W3-virus, and (2) a BDD-aM1-BSA electrode for measurement of charge transfer resistance of the sample with the M1 protein of W5-virus. The electrodes: (1) BDD-aM1-BSA and (2) BDD-aM1-BSA were incubated with prepared samples of viruses W3 and W5, respectively and after suitable incubation, the electrodes ((1) BDD-aM1-BSA-W3, (2) BDD-aM1-BSA-W5), were immersed in separate electrolytes of 1 mM $K_3Fe(CN)6$ in 0,1 PBS. The measurements were conducted separately, but in the same conditions. The procedure was performed at optimized conditions: at a room temperature and incubation with virus samples (W3, W5) was performed for 5 minutes. The obtained results for W3 and W5 samples were compared with the results obtained for the blank exemplary electrodes: electrode (1) BDD-aM1-BSA: without anchored M1 protein, electrode (1) BDD-aM1-BSA-TRI: incubated with mixture of 0,5% Triton X-100/0,1 M PBS, without M1 protein, and (2) BDD-aM1-BSA: without M1 protein. Fig. 8 presents a graph with obtained values of charge transfer resistance for the corresponding samples (W3 and W5 and reference samples), wherein the vertical error bars show the standard deviations of measurements taken from three independent experiments; and Fig. 9 presents a table with collected values of each element calculated from the EEQC obtained from fitting procedure.

Almost no change was observed upon incubation of the blank exemplary electrode (BDD-aM1-BSA-TRI) with the mixture of 0,5% Triton X-100/0,1 PBS, which indicates that using of the mixture does not affect the shape of EIS spectra. Therefore, the mixture of 0,5% Triton X-100/0,1 PBS may be used for extracting the M1 protein out of the cells of the virus. Moreover the mixture may be in contact with the sensing surface of the modified BDD electrode without affecting the results of the measurement. Comparison of the results obtained for the reference electrode (incubated with reference sample - without M1 protein) with the results obtained for electrodes with anchored M1 protein for both: W3 and W5 samples presents remarkable change in charge transfer resistance. For the M1 protein originating from W3 sample, the $R_{ct}$ value increased by 83% as compared with its value before the incubation. For the M1 protein originating from W5 sample, the $R_{ct}$ value increased by 98%, as compared with its value before the incubation.

The significant difference in $R_{ct}$ values: before and after incubation for electrodes with anchored M1 proteins (W3, W5), as well as the observed slight change in the $R_{ct}$ value - for blank electrodes (in control measurement), indicate that the registered changes are caused only by the specific protein binding (formation of probe-analyte complexes on the BDD modified surface), and not by any unspecific binding or electrode instability or measurement error.

The remarkable change in charge transfer resistance ($R_{ct}$) that has been observed in the above described experiments for the modified BDD electrodes after incubation with real virus sample (W3, W5), as well as a lack of any specific response towards pure solutions of 0,1 M PBS, 0,5% Triton X-100/0,1 PBS or biological sample originating from a healthy organism, have confirmed that the sensor according to the present disclosure - with the modified BDD electrode, is capable for carrying out detection assays that can reveal the presence of various biological molecules (analytes) in the biological samples, such as of the M1 protein of influenza virus. Moreover, the EIS sensor comprising the modified BDD electrode enables detecting a biomolecule even in the solution of a trace concentration of the analyte (e.g. the M1 protein), thereby providing an efficient method for recognizing various biological molecules, and therefore recognizing various diseases, such as viral disease, at a very early stage of infection.

The tests that have been performed confirmed the specificity and universality of the used sensor (comprising the BDD electrode modified with probe biomolecules), as well as the sensitivity of the sensor. The EIS sensor is capable of detecting various biological molecules including the influenza virus in a sample of concentration of the M1 protein at the level of 10 fg/mL. The tests results were repetitive for a set of prepared electrodes.

The EIS sensor enables detection of different analyte biomolecules, such as amino acids, hormones, sugars, nucleic acids, peptides or proteins, such as for example M1 protein of influenza virus, in both: a sample of small volume and a sample of extremely low concentration of the analyte biomolecules. Moreover, the EIS sensor provides a short and easy detection procedure, taking into account a short time of incubation (approximately 5 minutes) and mild conditions of the measurement (room temperature).

Moreover, the obtained organic layer of diazonium salt of aminobenzoic acid, exemplary treated with BSA, provides unexpectedly an improved saturation of the various probes, which was demonstrated herein by investigation of the anti-M1 protein. When the M1 proteins anchor to the corresponding probes (anti-M1 protein) immobilized on the organic layer of BDD surface, there is observed a blockage of the electrons transfer between electrode and redox active species ($Fe^{2+}/Fe^{3+}$) present in electrolyte (1 mM $K_3Fe(CN)_6$ + 0,1 PBS) which can be seen as an increase in $R_{ct}$ value, that confirms the presence of the influenza virus in the investigated sample. The same phenomenon is observed in case of investigation of different biological molecules (analyte), when providing the modified BDD substrate with immobilized corresponding probe biomolecules, i.e. the probe molecules that are able to interact with the analyte biomolecules, with formation of probe-analyte complexes during suitable incubation procedure.

The method according to the present disclosure provides a short detection time, very high sensitivity (e.g. below 10 fg/mL of the M1 protein in sample), short incubation time (only 5 minutes) at room temperature, lack of unspecific response towards both: a buffer solution and a sample received from healthy patient and universality of influenza detection, and therefore it may be used in detection, for instance, the presence of influenza virus in a swab at a very early stage of infection.

Moreover, the method according to the present disclosure, using the EIS technique, in comparison with other electrochemical methods, such as cyclic voltammetry or differential pulse voltammetry (which are also often used for characterization of molecular recognition), is less destructive.

Additionally, the high sensitivity of the method allows following the changes occurring on the electrode surface after each step of its modification, as well as during detection procedure.

Moreover, the obtained organic layer of diazonium salt of aminobenzoic acid treated with BSA provides improvement in saturation of various probe biomolecules, such as for example the anti-M1 protein. Therefore, when the analyte biomolecules, such as the M1 proteins, anchor to the active spaces of the electrode surface, there can be observed the blockage of the electrons transfer between electrode and redox active species ($Fe^{2+}/Fe^{3+}$) present in electrolyte (1 mM $K_3Fe(CN)_6$ + 0,1 PBS), which is visible as an increase in $R_{ct}$ value, that confirms of presence of the influenza virus in the investigated sample.

EXAMPLE OF PREPARATION OF THE BDD SUBSTRATE

Si/BDD electrodes (substrates) were synthesized in an MW PA CVD system (Seki Technotron AX5200S, Japan) on p-type Si wafers with (100) orientation. The substrates were cleaned by sonication in acetone and propan-2-ol for 5 minutes in each solvent, respectively. Next, the substrates were seeded by sonication in a nanodiamond suspension (crystallite size of 5-10 nm) for 1 hour. Next, the substrates were dried under a stream of nitrogen. The temperature of the prepared substrates was kept at 700°C during the deposition process. During the first step of the deposition process, the substrates were etched in hydrogen plasma for 3 minutes. The excited plasma was ignited by microwave radiation (2,45 GHz). The plasma microwave power, optimized for diamond synthesis, was kept at 1300 W. The gas mixture ratio was of 1% of the molar ratio of $CH_4:H_2$ at a gas volume 300 sccm of the total flow rate. The base pressure was about $10^{-6}$ Torr and the process pressure was kept at 50 Torr. All samples were doped by using diborane ($B_2H_6$) dopant precursor; the [B]/[C] ratio was 10000 ppm in the plasma resulting in acceptor concentration of $3 \times 10^{21}$ cm$^{-3}$. The time of growing of polycrystalline layer was 6 hours, which resulted in obtainment of the deposited films of approx. 2 $\mu$m thick.

Next, the surface deposited BDD/Si electrodes was pre-treated in order to obtain an H-terminated surface and to etch $sp^2$ phase impurities. For all Si/BDD samples, the diamond surface was acidulated and next cleaned with hydrogen plasma. Next, the metallic impurities were dissolved in a hot aqua regia ($HNO_3$ : HCl /1:3), followed by removal of organic impurities by a hot "piranha" solution ($H_2O_2$ : $H_2SO_4$ / 1:3) at 90°C. Microwave hydrogen plasma treatment was performed using 1000 W microwave power and 300 sccm of hydrogen gas flow, for 10 minutes. As a result, predominantly hydrogen-terminated BDD substrates were obtained.

EXAMPLE OF PREPARATION OF THE MODIFIED BDD SUBSTRATE (MODIFICATION OF THE SURFACE OF BDD ELECTRODE WITH DIAZONIUM SALT OF 4-AMINOBENZOIC ACID)

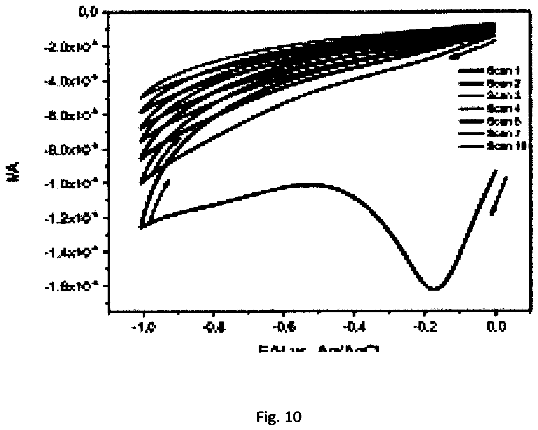

In order to treat the BDD surface with the diazonium salt of 4-aminobenzoic acid, the surface of each BDD substrate was sonicated for 5 minutes in ethanol. After the sonication, diazonium salt of 4-aminobenzoic was prepared as follows: aminobenzoic acid (10 mg, 0,073 mmol, Sigma-Aldrich) was dissolved in 1 ml of concentrated hydrochloric acid (POCh - Polish Chemicals, Poland), the reaction mixture was stirred and cooled in the ice bath to the temperature of 0°C. Next, 1 ml of water was added to the reaction mixture. After 10 minutes, a sodium nitrate (12,5 mg, 0,1811 mmol) was added into the stirred reaction mixture and dissolved. Next, the water (1,5 ml), in drops, was added into reaction mixture, and the mixture was stirred in the ice-water bath for 30 minutes. Next, 0,5 ml of obtained in reaction diazonium salt of 4-aminobenzoic acid was applied into electrochemical cell - onto BDD working electrode and the potential was cycled between 0 to -1,0 V for 10 scans using a scan rate of 100 mV/s. Treated electrodes were next rinsed with distilled water and methanol, and subsequently dried under a stream of air. The electrodes were subjected to investigation using cyclic voltammetry. In Fig. 10 there are presented the recorded cyclic voltammograms of reduction and deposition of diazonium salt of 4-aminobenzoic acid on the surface of BDD electrode which show the BDD electrode in contact of diazonium salt of 4-aminobenzoic acid in diluted HCl solution. In the first cycle, an irreversible reduction peak at -170 mV was observed, which may correspond to the electroreduction wave of the diazonium salt and the production of an aryl radicals, which in next step form a covalent bond onto the BDD electrode surface. In the next scans: from 2 to 10, a decreasing currents occurs, and in the last scan the reduction peaks are absent. This proves the surface saturation and suggests that a thin layer of benzoic acid functional groups was formed on the BDD surface.

EXAMPLE OF MODIFICATION OF THE BDD ELECTRODE WITH ANTI-M1 PROTEIN

(34,5 mg 0,18 mmol) of 1-ethyl-3-(3-dimethylaminopropyl)carbodiimide hydrochloride (EDC) and (20,9 mg 0,18 mmol) of N-hydroxysuccinimide (NHS) were dissolved in 5 ml of DMF and stirred for 10 minutes at a room temperature. 1 ml of this reaction mixture was applied onto the BDD electrode surface that has been previously treated with the diazonium salt of 4-aminobenzoic acid, and in the next step the solution of an antibody of a concentration of 6 μg/ml (diluted in a phosphate buffer of pH = 5,5) was applied onto the electrode and left for 24 hours, in 4°C. The modified BDD electrode was washed with distilled water and methanol, and next dried under a stream of air.

FIRST EMBODIMENT OF DETECTION OF THE INFLUENZA VIRUS IN THE BIOLOGICAL SAMPLE

**I Production of recombinant M1 in the bacterial expression system**

The M1 gene of Influenza A virus A/England/195/2009 (H1N1) was PCR amplified using plasmid DNA (pPol-M1) as a template and oligonucleotides (For-M1-BamHI 5' TTGGATCCAGTCTTCTAACCGAGGTCGAA 3' and Rev-M1-EcoRI 5' TTTGAATTC CTTGAATCGCTGCATCTGC 3') as primers. The obtained PCR product of 770 bp was digested with BamHI and EcoRI enzymes and cloned into a commercial vector, pGEX 2TK (GE Healthcare). The resulting plasmid, pM1-GST was verified by restriction analysis and nucleotide sequencing. pM1-GST was used to transform BL21 E. coli strain, and the recombinant strain was used to overproduce M1-GST after addition of IPTG (final concentration 1 MM). The 54 kDa protein was visualized on a blue-coomassie-stained gel and purified by affinity chromatography on glutathione resin (GE Helathcare). 0,2 mg of pure M1-GST protein was obtained from 0,25 liter of culture.

**II Production of polyclonal anti-M1 antibodies**

For antibody production, a rabbit was immunized intraperitoneally with 300 μg of purified M1-GST protein mixed with incomplete Freund's adjuvant in a total volume of 1 ml. The injections took place on days 0 and 21. On day 42, blood was collected. The obtained sera was tested for purified protein and optimal dilution of anti-M1 sera for western blot analysis which was established as 1:10,000. The universality and selectivity of the antibodies were confirmed using western blot. Anti-M1 antibodies were purified by affinity chromatography on CNBr-activated Sepharose 4B coupled with M1-His protein. It was used for fabrication of biosensor.

**III Propagation of influenza virus**

Avian influenza A/ostrich/Denmark/96-72420/96 (H5N2), A/GraylagGoose/Poland/MW74/10 (H5N2), A/Afri.Star./Eng-Q/938/79 (H7N1) (from Department of Poultry Diseases, National Veterinary Research Institute, Pulawy, Poland) and human influenza A viruses (pandemic H1N1 strains) (from the collection of the Department of Recombinant Vaccines, University of Gdansk, Poland) were propagated in Madin-Darby canine kidney cells (MDCK) cultured at 37°C under 5% $CO_2$ in Dulbecco's Modified Eagle's Medium (D-MEM) (Sigma-Aldrich), supplemented with 2 mM L-glutamine, 0,2% bovine serum albumin, 25 mM HEPES buffer, 100 U/ml of penicillin, 100 μg/ml of streptomycin in the presence of 2 μg/ml TPCK (L-1-Tosylamide-2-phenylethyl chloromethyl ketone) - trypsin (Sigma-Aldrich). Viral stocks were stored at -70°C and titrated by plaque assay before use.

**IV Detection of the M1 protein of influenza virus**

For the non-modified and modified BDD electrodes, cyclic voltammetry (CV) and electrochemical impedance spectroscopy (EIS) studies were performed, using an AutoLab PGStat 302N potentionstat-galvanostat system (Methrom, Autolab) in the standard three-electrode assembly, where the BDD electrode was serving as a working electrode. The active area of the electrode surface has a circular shape with a diameter of 4 mm (geometric surface area of 0,1256 $cm^2$). A Pt wire was used as a counter electrode, while Ag/AgCl/0,1 M KCl was used as a reference electrode. All electrochemical tests were carried out in 1 mM $K_3Fe(CN)_6$ / 0,1 M PBS that was previously deaerated. Next, the BDD electrode previously treated with the diazonium salt of 4-aminobenzoic acid was incubated with the anti-M1 protein obtained in step II above (Production of polyclonal anti-M1 antibodies). Subsequently, the electrode was incubated (1 hour, in 4°C) with 40 μL of 0,5% concentrated solution of bovine serum albumine (BSA) dissolved in 0,1 PBS solution. Subsequently the electrode was incubated with the sample solution prepared in step described above (Propagation of influenza virus). After incubation, the electrode was rinsed with a fresh 0,1 M PBD, and subsequently the surface of the electrode was immersed with an electrolyte solution of ($K_3Fe(CN)_6$ / 0,1 M PBS, 100 μL). The whole experimental setup was conditioned at a room temperature. The electrochemical measurements were initiated when the temperature of the electrode had reached equilibrium with external conditions. Subsequently, electrochemical impedance spectroscopy measurements were conducted at the frequency range of from 20 kHz to 1 Hz with 10 mV amplitude AC signal. The impedance spectra was collected at the formal potential of the redox reaction determined on the basis of redox peak position observed on the CV curves registered for a non-modified BDD electrode. Before each spectra registration, the potential was held for 1 minute to achieve a steady state condition. After each electrochemical measurement, conducted for each sample the electrode surface was washed once again by a fresh 0,1 PBS. The same procedure was carried out for each virus sample obtained in step III - described above.

EXEMPLARY, NOT BEING PART OF THE INVETION, DETECTION OF THE INFLUENZA VIRUS IN THE BIOLOGICAL SAMPLE

## I Control measurements

In order to exclude the electrode (BDD-aM1-BSA) response to any unspecific detection of the electrode behavior, the BDD electrode modified with the anti-M protein (BDD-aM1-BSA) was tested after substrate incubation in 0,1 PBS and the biological material present in the oral cavity of healthy patient was used as a blank sample. First, a BDD-aM1-BSA electrode was incubated with 0,1 PBS for 30 minutes, 4°C and after reaching room temperature EIS spectra was registered. Then, the electrode was washed with a fresh 0,1 PBS and incubated for 30 minutes at 4°C in 40 $\mu$L of diluted in 0,1 PBS throat swab from a healthy patient. Finally, the electrode response was registered as impedance spectra.

## II Detection of the M1 protein

To analyze the sensitivity of the electrode, the BDD-aM1-BSA electrode was incubated with a serial dilution of the M1 protein. According to the preliminary studies, as a first step BDD-aM1-BSA electrode was incubated with 40 $\mu$L of 10 fg/mL M1 protein solution for 30 minutes, at 4°C. After incubation, the electrode surface was washed with a fresh 0,1 PBS and immersed in deaerated 1 mM $K_3Fe(CN)_6$ / 0,1 PBS. When electrode had reached room temperature, the EIS spectrum was recorded. Next, the electrode surface was rinsed with 0,1 PBS and then the electrode was incubated with 40 $\mu$L of the M1 solution containing 40 fg/mL of M1 protein. Thus, the electrode was exposed to 50 fg/mL of protein molecules in total. After 30 minutes of incubation, at 4°C, the whole experimental procedure: washing with 0,1 PBS, the EIS measurement in ferricyanide solution was repeated. In the next step, after subsequent electrode incubation in 40 $\mu$L solution of 50 fg/mL concentrated M1 protein and finally with 8 pg/mL concentrated M1 protein, the electrochemical detection was carried out. Therefore, in the last two steps the total concentration of the M1 loaded on the electrode surface was 100 fg/mL and 8,1 pg/mL, respectively.

## III Detection of the M1 protein from virus suspension

In order to exclude any impact on the electrode response resulting from the presence of Triton X-100, the BDD-aM1-BSA electrode was incubated in 40 $\mu$L of 0,5% Triton X-100 in 0,1 PBS for 30 minutes at a room temperature. Next, the electrode was washed with 0,1 PBS and EIS spectra was recorded in the ferricaynide redox solution. The same electrode, after rinsing with 0,1 PBS was incubated for 5 minutes with 40 $\mu$L of W3 virus. Another BDD-aM1-BSA electrode was used for M1 protein released from W5 virus suspended in 0,5% Triton X-100/0,1 PBS solution. Before incubation, the solution of W3 virus or W5 virus was vigorously shaken. For each measurement the EIS spectra were taken and evaluated.

## Claims

1.  A modified BDD (boron doped diamond) substrate comprising an organic layer comprising carboxyl groups, where the organic layer on the BDD substrate comprises organic groups perpendicularly attached to the surface of the substrate, wherein organic groups have been electrochemically immobilized from diazonium salt of 4-aminobenzoic acid in an AC scanning CV (Cyclic Voltammetry) process, and the substrate further comprises probe molecules covalently bound to the carboxyl groups of the organic layer.

2.  The substrate according to claim 1, wherein the AC scanning signal has a negative potential of up to -1 V and a scanning rate of 50 to 200 mV/s wherein the organic layer is formed in the CV electrochemical cell comprising a silver-chloride (Ag/AgCl) electrode as a reference electrode and a platinum electrode as a counter electrode and/or the organic layer is formed by applying the scanning signal in a plurality of cycles, preferably 10 cycles

3.  The substrate according to any of claims 1-2, wherein the modified BDD substrate comprises BSA molecules bound to the carboxyl groups of the organic layer.

4. The substrate according to any of claims 1-3, wherein the modified BDD substrate further comprises analyte molecules bound to probe molecules wherein the modified BDD substrate comprises, as the analyte molecules, at least one molecule selected from the group consisting of: amino acids, hormones, sugars, nucleic acids, peptides and proteins and/ or the modified BDD substrate comprises, as the probe molecules, anti-M1 proteins of influenza virus or the modified BDD substrate comprises, as the analyte molecules, M1 proteins of influenza virus.

5. An impedance sensor for detecting biological molecules, comprising an EIS (Electrochemical Impedance Spectroscopy) meter, wherein the sensor comprises, as a working electrode, the modified BDD substrate according to any of claims 1-4.

6. The sensor according to claim 8 , comprising, as an electrolyte, a solution of 1 mM $K_3Fe(CN)_6$ / 0,1 PBS and as a reference electrode, a chloro-silver electrode: Ag/AgCl/0,1 M KCl and as a counter electrode, a platinum (Pt) electrode.

7. A method for detecting biological molecules, the method comprising the steps of:

- providing the sensor according to any of claims 5-6;
- incubating the working electrode of the sensor with a diluted biological sample;
- immersing the working electrode of the sensor in an electrolyte of the EIS cell;
- conducting an EIS measurement at an AC current to register EIS measurement results; and
- comparing the EIS measurement results with reference EIS data obtained for a sample without analyte biomolecules.

8. The method according to claim 7, comprising using an anti-M1 protein of an influenza virus as the probe biomolecule or analyte biomolecule.


**Patentansprüche**

1. Modifiziertes BDD-(bordotierter Diamant)-Substrat, umfassend eine organische Schicht, die Carboxylgruppen umfasst, wobei die organische Schicht auf dem BDD-Substrat organische Gruppen umfasst, die an die Oberfläche des Substrats perpendikulär angeheftet sind, wobei die organischen Gruppen aus Diazoniumsalz von 4-Aminobenzoesäure in einem AC-Abtastungs-CV-(Cyclovoltammetrie)-Prozess elektrochemisch immobilisiert wurden und das Substrat ferner Sondenmoleküle umfasst, die an die Carboxylgruppen der organischen Schicht kovalent gebunden sind.

2. Substrat nach Anspruch 1, wobei das AC-Abtastsignal ein negatives Potential von bis zu -1 V und eine Abtastrate von 50 bis 200 mV/s aufweist, wobei die organische Schicht in der elektrochemischen CV-Zelle gebildet ist, die eine Silberchlorid-(Ag/AgCl-)-Elektrode als eine Referenzelektrode und eine Platin-Elektrode als eine Gegenelektrode umfasst und/oder die organische Schicht gebildet ist durch Anlegen des Abtastsignals in einer Vielzahl von Zyklen, vorzugsweise 10 Zyklen.

3. Substrat nach einem der Ansprüche 1-2, wobei das modifizierte BDD-Substrat BSA-Moleküle umfasst, die an die Carboxylgruppen der organischen Schicht gebunden sind.

4. Substrat nach einem der Ansprüche 1-3, wobei das modifizierte BDD-Substrat ferner Analyt-Moleküle umfasst, die an Sondenmoleküle gebunden sind, wobei das modifizierte BDD-Substrat, als die Analyt-Moleküle, zumindest ein Molekül umfasst, ausgewählt aus der Gruppe umfassend: Aminosäuren, Hormone, Zucker, Nukleinsäuren, Peptide und Proteine, und/oder das modifizierte BDD-Substrat, als die Sondenmoleküle, Anti-M1-Proteine des Influenzavirus umfasst oder das modifizierte BDD-Substrat, als die Analytmoleküle, M1-Proteine des Influenzavirus umfasst.

5. Impedanzsensor zum Detektieren von biologischen Molekülen, umfassend ein EIS-(Elektrochemische Impedanzspektroskopie)-Messgerät, wobei der Sensor als eine Arbeitselektrode das modifizierte BDD-Substrat nach einem der Ansprüche 1-4 umfasst.

**6.** Sensor nach Anspruch 8,
umfassend, als ein Elektrolyt, eine Lösung von 1 mM K$_3$Fe(CN)$_6$/0.1 PBS und, als eine Referenzelektrode, eine Chlor-Silber-Elektrode: Ag/AgCl/0.1 M KCl und, als eine Gegenelektrode, eine Platin-(Pt)-Elektrode.

**7.** Verfahren zum Detektieren von biologischen Molekülen, wobei das Verfahren die Schritte umfasst:

- Bereitstellen des Sensors nach einem der Ansprüche 5-6;
- Inkubieren der Arbeitselektrode des Sensors mit einer verdünnten biologischen Probe;
- Eintauchen der Arbeitselektrode des Sensors in einen Elektrolyten der EIS-Zelle;
- Durchführen einer EIS-Messung mit einem Wechselstrom, um EIS-Messungsergebnisse zu registrieren; und
- Vergleichen der EIS-Messungsergebnisse mit Referenz-EIS-Daten, die für eine Probe ohne Analyt-Biomoleküle erhalten wurde.

**8.** Verfahren nach Anspruch 7,
umfassend die Verwendung eines Anti-M1-Proteins eines Influenza-Virus als das Sonden-Biomolekül oder Analyt-Biomolekül.

## Revendications

**1.** Substrat BDD (diamant dopé au bore) modifié comprenant une couche organique comprenant des groupes carboxyle, la couche organique sur le substrat BDD comprenant des groupes organiques liés perpendiculairement à la surface du substrat, dans lequel les groupes organiques ont été immobilisés par voie électrochimique à partir d'un sel de diazonium d'acide 4-aminobenzoïque dans un procédé de VC (voltampérométrie cyclique) à balayage en CA, et le substrat comprend en outre des molécules de sonde liées par liaison covalente aux groupes carboxyle de la couche organique.

**2.** Substrat selon la revendication 1, dans lequel le signal de balayage en CA présente un potentiel négatif allant jusqu'à -1 V et une vitesse de balayage de 50 à 200 mV/s dans lequel la couche organique est formée dans la cellule électrochimique VC comprenant une électrode en chlorure d'argent (Ag/AgCl) en tant qu'électrode de référence et une électrode en platine en tant que contre-électrode et/ou la couche organique est formée par l'application du signal de balayage dans une pluralité de cycles, de préférence 10 cycles.

**3.** Substrat selon l'une quelconque des revendications 1 et 2, dans lequel le substrat BDD modifié comprend des molécules de BSA liées aux groupes carboxyle de la couche organique.

**4.** Substrat selon l'une quelconque des revendications 1 à 3, dans lequel le substrat BDD modifié comprend en outre des molécules d'analyte liées à des molécules de sonde dans lequel le substrat BDD modifié comprend, en tant que molécules d'analyte, au moins une molécule sélectionnée dans le groupe constitué : des acides aminés, des hormones, des sucres, des acides nucléiques, des peptides et des protéines et/ou le substrat BDD modifié comprend, en tant que molécules de sonde, des anti-protéines M1 de virus de la grippe ou le substrat BDD modifié comprend, en tant que molécules d'analyte, des protéines M1 de virus de la grippe.

**5.** Capteur d'impédance pour détecter des molécules biologiques, comprenant un appareil de mesure SIE (spectroscopie d'impédance électrochimique), dans lequel le capteur comprend, en tant qu'électrode de travail, le substrat BDD modifié selon l'une quelconque des revendications 1 à 4.

**6.** Capteur selon la revendication 8, comprenant, en tant qu'électrolyte, une solution de 1 mM de K$_3$Fe(CN)$_6$/0,1 de PBS et en tant qu'électrode de référence, une électrode en chlorure d'argent: Ag/AgCl/0,1 M de KCl et en tant que contre-électrode, une électrode en platine (Pt).

**7.** Procédé de détection de molécules biologiques, le procédé comprenant les étapes suivantes :

- la fourniture du capteur selon l'une quelconque des revendications 5 et 6 ;
- la mise en incubation de l'électrode de travail du capteur avec un échantillon biologique dilué ;
- l'immersion de l'électrode de travail du capteur dans un électrolyte de la cellule SIE ;
- la mise en œuvre d'une mesure SIE à un courant alternatif pour enregistrer des résultats de mesure SIE ; et
- la comparaison des résultats de mesure SIE à des données SIE de référence obtenues pour un échantillon sans biomolécules d'analyte.

8. Procédé selon la revendication 7, comprenant l'utilisation d'une anti-protéine M1 d'un virus de la grippe comme biomolécule de sonde ou biomolécule d'analyte.

Fig . 1

Fig. 2

Fig. 3

Fig. 4

| sample | Re / Ω | $Q_{dl}$ / μΩ⁻¹sⁿ | n | $R_{ct}$ / 10⁵ Ω | $A_W$ / Ω s⁰·⁵ |
|---|---|---|---|---|---|
| BDD | 379.0 | 1.310 | 0.936 | 0.23 | 3167 |
| BDD-aM1 | 388.9 | 1.329 | 0.943 | 1.29 | – |
| BDD-aM1-BSA | 391.7 | 1.182 | 0.944 | 2.13 | – |
| BDD-aM1-BSA-PBS | 367.6 | 1.139 | 0.946 | 2.26 | – |
| BDD-aM1-BSA-BIOL | 386.6 | 1.168 | 0.947 | 2.34 | , |

Fig. 5

Fig. 6

| sample | $R_s / \Omega$ | $Q_{dl} / \mu F \Omega^{-1} s^n$ | n | $R_{ct} / 10^5 \Omega$ |
|---|---|---|---|---|
| BDD-aM1-BSA | 391.7 | 1.18 | 0.944 | 2.13 |
| BDD-aM1-BSA-10 fg/mL | 325.9 | 1.15 | 0.948 | 3.20 |
| BDD-aM1-BSA-50 fg/mL | 389.5 | 1.13 | 0.955 | 4.28 |
| BDD-aM1-BSA-100 fg/mL | 327.0 | 1.11 | 0.952 | 5.12 |
| BDD-aM1-BSA-8 pg/mL | 358.6 | 1.09 | 0.953 | 5.24 |

Fig. 7

Fig. 8

| sample | R1 / Ω | CPE / µFΩ⁻¹s⁻ⁿ | n | R2 / 10⁵ Ω |
|---|---|---|---|---|
| (1) BDD-aM1-BSA | 337.4 | 1.00 | 0.956 | 2.37 |
| (1) BDD-aM1-BSA-TRI | 332.5 | 1.03 | 0.952 | 2.44 |
| (1) BDD-aM1-BSA-W3 | 377.25 | 0.90 | 0.951 | 4.33 |
| (2) BDD-aM1-BSA | 387.26 | 1.07 | 0.947 | 2.46 |
| (2) BDD-aM1-BSA-W5 | 326.98 | 0.92 | 0.954 | 4.88 |

Fig. 9

Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014014371 A

**Non-patent literature cited in the description**

- **CARLISLE, J. A.** Diamond films: Precious biosensors. *Nat. Mater.,* 2004, vol. 3, 668-669
- **BOGDANOWICZ, R. et al.** Influence of the boron doping level on the electrochemical oxidation of the azo dyes at Si/BDD thin film electrodes. *Diam. Relat. Mater.,* 2013, vol. 39, 82-88
- **PRADO, C. et al.** Electrochemical analysis of nucleic acids at boron-doped diamond electrodes. *Analyst,* 2002, vol. 127, 329-332
- **ANCHANA PREECHAWORAPUN et al.** Development of Amperometric ImmunosensorUsing Boron-Doped Diamond with poly(o-aminobenzoic acid. *Anal. Chem.,* 2008, vol. 80, 2077-2083
- **DA-JUNG CHUNG et al.** Doping Level of Boron-Doped Diamond Electrodes Controls the Grafting Density of Functional Groups for DNA Assays. *Electrochimica Acta,* 2012, vol. 76, 394-403
- **AMANI CHROUDA et al.** Electrically addressable deposition of diazonium- functionalized antibodies on boron-doped diamond microcells for the detection of Ochratoxin A. *Anal. Methods,* 2015, vol. 7, 2444-2451
- **(LUBOMÍR ŠVORC et al.** Doping Level of Boron-Doped Diamond Electrodes Controls the Grafting Density of Functional Groups for DNA Assays. *Appl. Mater. Interfaces,* 2015, vol. 7, 18949-1895
- **SUNIL K. ARYA et al.** Label free biosensor for sensitive human influenza virus hemagglutinin specific antibody detection using coiled-coil peptide modified microelectrode array based platform. *Sensors and Actuators B,* 2014, vol. 194, 127-133
- **D. NIDZWORSKI et al.** Universal biosensor for detection of influenza virus. *Biosensors and Bioelectronics,* 15 September 2014, vol. 59, 239-242